Europäisches Patentamt

**European Patent Office**

Office européen des brevets

(19)

(11) Numéro de publication: **0 027 249**
**B1**

# (12) FASCICULE DE BREVET EUROPEEN

(45) Date de publication du fascicule du brevet:
**17.08.83**

(51) Int. Cl.³: **A 61 K 39/145, C 12 N 7/00**

(21) Numéro de dépôt: **80106094.8**

(22) Date de dépôt: **08.10.80**

(54) **Procédé de préparation de souches vaccinales de virus de l'influenza de type A, souche vaccinale et vaccin la contenant.**

(30) Priorité: **16.10.79 US 85437**

(43) Date de publication de la demande:
**22.04.81 Bulletin 81/16**

(45) Mention de la délivrance du brevet:
**17.08.83 Bulletin 83/33**

(84) Etats contractants désignés:
**AT BE CH DE FR GB IT LI LU NL SE**

(56) Documents cités:
**EP-A-0 007 467**
**FR-A-2 245 376**
**FR-A-2 253 535**
**FR-A-2 354 778**
**US-A-3 992 522**
**BULLETIN DE L'ORGANISATION MONDIALE DE LA SANTE, vol. 41, pages 643—645 (1969) E. D. KILBOURNE: »Future Influenza Vaccines and the Use of Genetic Recombinants«**
**THE LANCET, 11 décembre 1971, pages 1271—1273 A. S. BEARE et al.: »Recombinant Influenza-A Viruses as Live Vaccines for Man«**

(73) Titulaire: **Smith Kline - RIT Société anonyme dite:, rue du Tilleul, 13, B-1320 Genval (Rixensart) (BE)**

(72) Inventeur: **Löbmann, Michèle, rue d'Angoussart, 165, B-1301 Bierges (Wavre) (BE)**
Inventeur: **Florent, Gérard, Champ du Cygne, 13, B-1320 Genval (Rixensart) (BE)**

(74) Mandataire: **Tasset, Gérard, Smith Kline - RIT rue de l'Institut, 89, B-1330 Rixensart (BE)**

Procédé de préparation de souches vaccinales de virus de l'influenza de type A,
souches vaccinales obtenues par ce procédé et vaccins les contenant

La présente invention est relative à un procédé de préparation de nouvelles souches atténuées de virus grippal de type A, aux souches vaccinales obtenues par ce procédé et aux vaccins les contenant.

Jusqu'à présent, différentes techniques ont été utilisées pour atténuer le virus grippal en vue de la préparation de vaccins antigrippaux.

Une de ces techniques comprend la recombinaison d'une souche pathogène avec une souche virale antigéniquement distincte et connue pour être atténuée pour l'homme, suivie par l'isolement d'un recombinant atténué adéquat.

Un exemple de vaccin obtenu par un procédé impliquant une recombinaison virale et l'isolement d'un recombinant atténué adéquat est donné par le brevet N° 3 953 592 des Etats Unis d'Amérique.

Il est bien connu qu'un problème particulier pour l'immunisation contre le virus grippal de type A provient du fait que, presque chaque année, le sérotype de la souche de virus grippal circulant dans le monde diffère légèrement du sérotype des souches précédemment observées. Etant donné que, pour être efficace, un antigène vaccinal doit être le plus proche possible de celui de la souche dominante, un vaccin antigrippal vivant doit être périodiquement ajusté.

La recombinaison entre une souche virulente circulante de virus grippal de type A et récemment isolée et une souche atténuée bien connue (par exemple la souche de virus grippal A/PR/8/34) constitue un moyen remarquable pour l'ajustement rapide de souches de virus grippal grâce au fait que, par recombinaison, les propriétés d'une souche peuvent être, en un temps très court, transférées à une autre souche.

Cependant, la recombinaison entraîne la formation d'une grande variété de recombinants dont très peu conviennent à une utilisation vaccinale. En effet, ces derniers doivent présenter un certain nombre de caractéristiques telles que sérotype du virus sauvage, atténuation, immunogénicité, non transmissibilité aux personnes en contact avec les vaccinés, stabilité génétique, capacité de croissance suffisante et bonne stabilité en cours de stockage.

A ce jour, on a développé différents marqueurs pour la sélection de souches de virus grippal de type A, lesquelles constituent alors des candidats possibles pour la préparation de vaccins vivants.

Comme exemples de marqueurs, on peut citer la résistance de la souche aux inhibiteurs présents dans le sérum normal (comme dans le brevet n° 3 953 592 des Etats-Unis d'Amérique) et la proportion du génome donné par chaque souche parente et exprimée en pourcentage d'hybridisation RNA − RNA (G. Florent dans Developments in Biological Standardization, Vol 39, 11 − 14, S. KARGER, Basel, 1977).

Nous avons maintenant trouvé un marqueur biochimique nouveau et fiable de l'atténuation d'un virus grippal recombinant pour son administration à des êtres humains. Contrairement aux marqueurs précédemment connus, le présent marqueur est basé sur la structure biochimique particulière du virus et constitue donc l'expression d'une relation structure/activité. Nous avons en effet trouvé que, dans le cas d'un virus grippal recombinant de type A, une constellation hétérogène des gènes codant pour les polymérases (P) — c'est-à-dire lorsque les gènes codant pour les polymérases (P) ne sont pas tous issus du même parent, en l'occurrence deux gènes provenant d'un parent et un gène provenant de l'autre parent — constitue un marqueur d'atténuation suffisante pour une utilisation vaccinale chez les êtres humains.

Avant l'invention, des études comparatives avaient déjà été effectuées sur la réassortiment de gènes par recombinaison de virus grippaux mais aucune évidence de corrélation n'avait été trouvée entre la composition du génome des recombinants et leur utilisation chez l'homme (J. S. Oxford et al., Nature, 273, 778 − 779, 1978).

La présente invention concerne la production d'une souche recombinante de virus grippal de type A utilisable pour la préparation d'un vaccin antigrippal par recombinaison d'une souche atténuée de virus grippal de type A (plus particulièrement la souche A/PR/8/34 avec une souche pathogène également de type A (plus particulièrement la souche A/California: 10/78) et isolement d'une souche recombinante dont le sérotype est celui de la souche pathogène parente caractérisé en ce que la progéniture résultant de la recombinaison et présentant le sérotype de la souche pathogène est clonée, qu'on analyse les clones au point de vue du réassortiment de leur gènes codant pour les polymérases (P) et qu'on isole comme souche vaccinale un recombinant dont les gènes codant pour les polymérases (P) ne sont pas tous issus du même virus parent.

Par recombinaison de la souche atténuée de virus grippal A/PR/8/34 ($H_0N_1$), laquelle présente une forte capacité de croissance, avec la souche pathogène de virus grippal A/California/10/78 ($H_1N_1$), nous avons pu isoler, parmi les différents recombinants résultant, une nouvelle souche de virus grippal qui a été dénommée RIT 4265, qui a le sérotype $H_1N_1$ et qui est de valeur pour la production d'un vaccin vivant contre la souche de virus grippal A/California/10/78 et les souches comparables, ladite nouvelle souche de virus grippal présentant, en ce qui concerne ses gènes codant pour les polymérases (P), un réassortiment de gènes qui ne sont pas tous issus du même parent: deux gènes provenant de la souche A/PR/8/34 et un gène provenant de la souche A/California/10/78.

La souche de virus grippal A/California/10/78 est une souche sauvage isolée d'un patient de Californie. Elle a été reçue du W. H. O. Collaborating Center for Influenza, Atlanta, Georgia, U.S.A. à son troisième passage sur oeufs SPAFAS (SPAFAS Inc., Storrs, Connecticut, U.S.A.). Son sérotype est identique à celui de la souche prototype A/Brazyl/11/78 (H₁N₁). La souche de virus grippal A/California/10/78 a été déposée le 14 septembre 1979 dans la »Collection Nationale de Cultures de Microorganismes« (C.N.C.M.) de l'Institut Pasteur à Paris sous le N° C.N.C.M. I-098.

La souche de virus grippal RIT 4265 a également été déposée dans la même collection le 14 septembre 1979 sous le N° C.N.C.M. I-099.

Les souches C.N.C.M. I-098 et I-099 ci-avant mentionnées sont des souches de virus grippal H₁N₁. Chacune d'elles est formée de particules pléomorphiques à peu près sphériques présentant un diamètre extérieur d'environ 900 à 1000 Å et un noyau de 700 Å. La surface des particules virales de chacune de ces souches est recouverte de deux types de projections ou pointes d'environ 100 Å de longueur et possédant soit l'activité hémagglutinine soit l'activité neuraminidase du virus. L'antigène ribonucléoprotéine (RNP) issu de chacune des souches C.N.C.M. I-098 et I-099 présente une réaction croisée avec l'antisérum contre la RNP du virus grippal de type A.

La présente invention concerne donc également la nouvelle souche de virus grippal de type A déposée dans la collection Nationale de Cultures de Micro organismes de l'Institut Pasteur à Paris sous le numéro C.N.C.M. I-099 et le vaccin antigrippal comprenant une dose efficace de cette souche de virus grippal avec un diluant pharmaceutique pour administration nasale.

La souche de virus grippal de type A C.N.C.M. I-099 est un recombinant obtenu en mélangeant des quantités aliquotes des souches A/PR/8/34 et C.N.C.M. I-098, en laissant le mélange au repos pendant quelques heures à 4°C, en inoculant le mélange dans la cavité allantoïque d'oeufs fécondés de poule, en incubant les oeufs inoculés, en récoltant le matériel viral en le clonant par passages en dilution limite et en isolant un clone ayant une constellation hétérogène de ses gènes codant pour les polymérases (P).

Pour préparer un vaccin suivant l'invention, on cultive la souche recombinante de virus grippal de type A C.N.C.M. I-099 dans des oeufs fécondés, plus particulièrement dans la cavité allantoïque d'oeufs embryonnés de poule, suivant n'importe quelle technique bien connue dans la pratique de production de vaccins, pendant une durée suffisante pour permettre la production d'une importante quantité du dit virus, on récolte le matériel viral résultant et, si on le désire, on ajoute un stabilisant comme, par exemple, de la peptone, du saccharose ou tout autre stabilisant bien connu dans la technique. Le mélange est ensuite réparti dans des flacons en verre contenant des doses unitaires ou multiples de vaccin et la préparation est lyophilisée. Une dose efficace de vaccin contient, de préférence, au moins $10^7$ DIO₅₀ (dose infectieuse dans 50% des oeufs) de virus.

Les vaccins suivant l'invention sont administrés par voie nasale, le cas échéant après avoir été extemporanément reconstitués par addition d'eau ou de tout autre diluant ou composition pharmaceutique connu dans la technique pour la fabrication de préparations nasales sous forme de gouttes ou d'aérosol.

Pour assurer une réponse vaccinale optimale, l'administration du vaccin peut se faire par inoculation de deux doses unitaires successives, la seconde dose étant inoculée une semaine environ après la première.

L'invention est illustrée par les exemples qui suivent et dans lesquels il est fait référence à des oeufs; dans chaque cas, il s'agit d'oeufs provenant d'élevage SPF (specific pathogens free) conformes aux spécifications pour la production et le contrôle de vaccins viraux vivants aviaires, établies en 1976 par le Ministère de l'Agriculture, des Pêcheries et du Ravitaillement de Grande Bretagne.

Les exemples illustrent la présente invention sans en limiter la portée.

Exemple 1

Un échantillon de 0,5 ml d'une suspension reconstituée de la souche de virus grippal A/PR/8/34 lyophilisée contenant $10^{9,2}$ DIO₅₀ par millilitre est mélangé à un échantillon de 0,5 ml de liquide allantoïdien d'oeufs embryonnés de poule contenant $10^{9,2}$ DIO₅₀ de la souche de virus grippal C.N.C.M. I-098 par millilitre et le mélange est maintenu à 4°C pendant quelques heures.

Le mélange est ensuite inoculé dans la cavité allantoïdienne d'oeufs embryonnés de poule, lesquels sont incubés à 35°C pendant 20 heures.

La progéniture de cette culture mixte est récoltée, diluée au dixième (v/v) et mélangée à un même volume de sérum de poule anti A/PR/8/34 traité à l'enzyme destructeur de récepteur (EDR) et dilué au cinquantième (v/v). Après une heure, le milieu est inoculé dans la cavité allantoïdienne d'oeufs embryonnés de poule lesquels sont alors incubés à 35°C pendant 24 heures. On effectue ensuite un autre passage dans les mêmes conditions mais pendant 48 heures.

Le virus obtenu est cloné dans la cavité allantoïdienne d'oeufs embryonnés de poule par passages en dilution limite, le premier passage étant effectué en présence de sérum de poule anti A/PR/8/34 traité au kaolin et dilué au centième (v/v), le deuxième passage étant effectué sans sérum et les deux passages suivants étant effectués en présence de sérum normal de cobaye.

Une souche ainsi isolée a été choisie, caractérisée et appelée RIT 4265. Elle a été déposée dans la

**0 027 249**

Collection Nationale de Cultures de Microorganismes (C.N.C.M.) de l'Institut Pasteur à Paris le 14 septembre 1979 sous le numéro C.N.C.M. I-099.

## Exemple 2

### Caractérisation du recombinant de virus grippal C.N.C.M. I-099

Le recombinant C.N.C.M. I-099 a été examiné au point de vue composition génotypique en employant une technique basée sur l'identification du RNA à double hélice formé par hybridisation entre le RNA complémentaire (c RNA) radioactif du recombinant et le RNA virionique non marqué des virus parents (A. HAY et al. dans Developments in Biological Standardization Vol. 39, 15 – 24, S. KARGER, Basel 1977), en procédant comme suit:

### Préparation du RNA complémentaire (c RNA) et du virion RNA (v RNA)

Des tapis monocellulaires de fibroblastes d'embryons de poulet sont infectés respectivement avec les souches virales A/PR/8/34, C.N.C.M. I-098 et C.N.C.M. I-099, en en employant au moins 2.000 unités hémagglutinantes de chaque virus.

Après une heure d'incubation à 36°C, de l'uridine marquée ($^3$H) est ajoutée à raison de 100 μCi par millilitre et, quatre heures après l'infection, le c RNA est isolé de l'extrait cytoplasmique comme décrit par A. HAY dans Virology, 83, 337 – 355, 1977.

Le RNA virionique est extrait du virus en employant le procédé au phénol/sulfate de dodécyle et de sodium décrit par C. SCHOLTISSEK dans Biochem. et Biophys. Acta 1979, 389 – 397, 1969.

### Hybridisation et électrophorèse sur gel

Chacune des préparations de c RNA est divisée en trois parties aliquotes. A la première on n'ajoute pas de v RNA tandis que, à la deuxième et à la troisième, on ajoute 10 microgrammes de v RNA de chacune des souches A/PR/8/34 et C.N.C.M. I-098. On ajoute neuf volumes de sulfoxyde de diméthyle et chaque mélange est incubé à 45°C pendant 30 minutes. On ajoute alors du chlorure de sodium, du chlorhydrate de trométhamine (pH 7,5) et de l'acide édétique pour atteindre respectivement une concentration $3 \times 10^{-2}$ M, $10^{-2}$ M et $1,5 \times 10^{-3}$ M. La concentration en sulfoxyde de diméthyle est réduite à 63% et la solution est incubée à 37°C pendant 12 heures.

Le RNA est précipité par deux volumes d'éthanol et redissous dans une solution tampon d'acétate de sodium ($10^{-2}$ M) à pH 4,5 additionnée de $ZnSO_4$ ($10^{-3}$ M). On ajoute 2500 u/ml de nucléase $S_1$ et, incubation à 37°C pendant quatre heures, le RNA est reprécipité et dissous dans un tampon composé d'urée (7 M), d'édétate de sodium ($5 \times 10^{-3}$ M) et d'acétate de tréthamine ($2 \times 10^{-2}$ M) à pH 7,8. Les RNAs à double hélice sont séparés par électrophorèse sur gel de polyacrylamide en plaque contenant de l'urée 7 M sous 80 volts pendant 16 heures et détectés par fluorographie, donnant pour le recombinant C.N.C.M. I-099 la composition génotypique suivante:

| Fragment RNA N° | | Origine |
|---|---|---|
| 1 | (codant pour les polymérases P) | A/PR/8/34 |
| 2 | (codant pour les polymérases P) | I-098 |
| 3 | (codant pour les polymérases P) | A/PR/8/34 |
| 4 | | I-098 |
| 5 | | A/PR/8/34 |
| 6 | | I-098 |
| 7 | | A/PR/8/34 |
| 8 | | A/PR/8/34 |

4

### Exemple 3

### Préparation du vaccin

Comme inoculum pour la préparation d'un lot d'ensemencement pour la production de vaccin, on utilise la souche C.N.C.M. I-099 obtenue à la fin du dernier passage de l'exemple 1.

Une quantité aliquote de la dite souche C.N.C.M. I-099 obtenue à la fin de l'exemple 1 est inoculée dans la cavité allantoïdienne d'oeufs fécondés de poule, lesquels sont ensuite incubés à 35°C pendant 2 à 3 jours.

Les liquides allantoïdiens contenant la souche C.N.C.M. I-099 sont récoltés, rassemblés et testés au point de vue stérilité et innocuité et on y ajoute de la peptone jusqu'à une concentration de 5% (v/v) en peptone.

La suspension de virus est répartie dans des flacons de 3 ml en verre, de manière à obtenir des doses unitaires (au moins $10^7$ $DIO_{50}$) de virus grippal et lyophilisée. Les flacons sont alors bouchés hermétiquement.

Pour l'administration, le vaccin est reconstitué extemporanément par addition de 0,5 ml d'un diluant qui peut, par exemple, être de l'eau distillée, du sérum physiologique ou une solution de saccharose à 5% (poids/volume) et on administre dans les narines le vaccin ainsi reconstitué.

### Exemple 4

### Vaccination avec le vaccin antigrippal atténué, souche C.N.C.M. I-099

### Matériel et méthode

Pour le test clinique, on a choisi 22 sujets âgés de 16 à 48 ans (moyenne d'âge: 25 ans) dont le taux d'anticorps déterminé par inhibition de l'hémagglutination était égal ou inférieur à 20 pour la souche C.N.C.M. I-099. A chaque sujet, on a administré une dose unitaire de vaccin contenant $10^{7,3}$ $DIO_{50}$ de la souche C.N.C.M. I-099 obtenue à la fin de l'exemple 3 et reconstitué immédiatement avant administration dans 0,5 ml d'une solution aqueuse stérile de saccharose à 5% (poids/volume), chaque sujet étant couché sur le dos pour l'administration de 5 gouttes de vaccin dans chaque narine.

Pour la détermination de la séroconversion (qui correspond soit à une élévation du taux d'anticorps par inhibition de l'hémagglutination de $<10$ à $\geq 10$ soit, lorsque le taux prévaccinal est $\geq 10$, à un quadruplement de ce taux d'anticorps), on a recueilli des échantillons de sang pour la détermination du taux d'anticorps par inhibition de l'hémagglutination pour la souche C.N.C.M. I-099 respectivement avant vaccination et 21 jours après vaccination. Les taux d'anticorps par inhibition de l'hémagglutination ont été déterminés en employant comme antigènes les souches A/Hong Kong/117/77, C.N.C.M. I-099 et A/Brazil/11/78.

Pour 14 sujets qui présentaient un taux d'anticorps $\leq 10$, on a également procédé à des lavages nasaux un jour avant vaccination et les premier, deuxième, troisième, cinquième et septième jour après vaccination. On a effectué un examen physique le jour de la vaccination (jour 0). Des listes de contrôle de symptômes et de températures corporelle ont été remplies journellement par tous les vaccinés.

Les sujets ont également été examinés pour l'apparition éventuelle des symptômes suivant: nez bouché, rhinorrhée, mal de gorge, enrouement, mal de tête, toux et expectoration.

### Résultats

### 1. Excrétion de virus

On a procédé à des lavages nasaux chez 14 sujets présentant un taux d'anticorps par inhibition de l'hémagglutination inférieur à 10 pour la souche C.N.C.M. I-099 (sauf le sujet N° 694 qui avait un taux de 10).

On n'a décelé aucune trace de virus hémagglutinant dans les lavages nasaux effectués le jour précédent la vaccination.

Les résultats individuels concernant l'excrétion de virus aux jours 1, 2, 3, 5 et 7 sont indiqués au Tableau 1.

Deux vaccinés ont excrété le virus vaccinal en quantité importante (supérieure à $10^3$ $DIO_{50}$ par 0,2 ml) respectivement au jour 2 (sujet N° 490) et aux jours 2 et 3 (sujet N° 698). Ces quantités ont ensuite diminué et plus aucun virus n'a été isolé au jour 5 chez le sujet N° 490 au jour 7 chez le sujet N° 698.

Un autre vacciné (sujet N° 693) a excrété le virus vaccinal du jour 1 au jour 3, mais il s'agissait de faibles quantités.

Sept autres vaccinés ont excrété le virus vaccinal au jour 1 seulement (dans le cas du N° 692, le virus a été isolé après deux passages sur oeufs) et un vacciné (le sujet n° 695) a excrété le virus au jour 2 seulement.

Tableau I

Excrétion de virus

| N° | Excrétion de virus (taux exprimé en log $DIO_{50}$/0,2 ml de lavage nasal) | | | | |
|----|------|------|------|------|------|
| | Jour 1 | 2 | 3 | 5 | 7 |
| 490 | NT | 4,5 | 2,5 | — | NT |
| 691 | — | — | — | — | — |
| 694 | — | — | — | — | NT |
| 698 | 1,6 | 3,25 | 3,0 | 0,25 | — |
| 703 | 1,0 | — | — | — | — |
| 705 | 0,5 | — | — | — | — |
| 707 | 0,5 | — | NT | NT | — |
| 712 | 0,2 | — | — | — | — |
| 713 | 1,3 | — | — | — | — |
| 715 | — | — | — | — | NT |
| 689 | 2,0 | — | — | — | — |
| 692 | + | — | — | — | — |
| 693 | 0,5 | 1,0 | 0,0 | — | — |
| 695 | — | 0,0 | — | — | — |

dans lequel:

»—«    indique »négatif«.
»+«    indique »positif au second passage«.
»NT«  indique »non testé«.

## 2. Sérologie

Le Tableau II donne les taux individuels en unités hémagglutinantes respectivement avant et 21 jours après vaccination. Chaque échantillon a été titré pour chacune des souches A/Hong Kong/117/77, C.N.C.M. I-099 et A/Brazil/11/78 (sauf dans le cas du sujet N° 662 dont le sérum n'a pas été testé pour la souche A/Hong Kong/117/77 après vaccination).

Treize parmi les 14 vaccinés qui ont été soumis au lavage nasal avaient un taux d'anticorps par inhibition de l'hémagglutination inférieur ou égal à 10 pour la souche C.N.C.M. I-099 et, parmi ces 14 sujets, onze ont séroconverti pour l'antigène vaccinal.

Un vacciné (le sujet N° 712) qui a excrété du virus vaccinal le jour 1 a séroconverti pour la souche A/Brazil/11/78 mais pas pour la souche A/Hong Kong/117/77 et le résultat pour la souche C.N.C.M. I-099 était à la limite du significatif.

Les deux vaccinés qui n'ont pas séroconverti pour les trois antigènes (les sujets N° 703 et 689) ont également excrété du virus, ce qui indique que tous les vaccinés ont été infectés.

Enfin, les huits vaccinés qui se sont révélés séropositifs pour les 3 antigènes avant vaccination et qui n'ont pas participé aux lavages nasaux ont séroconverti, à l'exception du sujet N° 704.

Les résultats de séroconversion sont résumés dans le Tableau III: ils indiquent que la moyenne géométrique des taux après vaccination était élevée pour les trois antigènes.

Tableau II

Taux en anticorps sériques chez les vaccinés

| N° | Taux en anticorps par inhibition de l'hémagglutination pour | | | | | |
| --- | --- | --- | --- | --- | --- | --- |
| | A/Hong Kong/117/77 | | CNCM N° I-099 | | A/Brazil/11/78 | |
| | Pre- | Post- | Pre- | Post- | Pre- | Post-vaccination |
| 490 | 5 | 160 | < 5 | 160 | <10 | 160 |
| 691 | 5 | 20 | 5 | 40 | 10 | 40 |
| 694 | 20 | 80 | 10 | 40 | 10 | 40 |
| 698 | < 5 | 80 | < 5 | 160 | <10 | 160 |
| 703 | < 5 | < 5 | < 5 | < 5 | <10 | < 10 |
| 705 | < 5 | 160 | < 5 | 40 | <10 | 160 |
| 707 | < 5 | 40 | < 5 | 40 | <10 | 160 |
| 712 | 5 | 5 | < 5 | 5 | <10 | 10 |
| 713 | 10 | 320 | < 5 | 320 | <10 | 160 |
| 715 | 10 | 160 | 5 | 40 | 10 | 80 |
| 689 | < 5 | < 5 | < 5 | < 5 | <10 | < 10 |
| 692 | 10 | 320 | < 5 | 160 | <10 | 160 |
| 693 | 10 | 320 | < 5 | 160 | <10 | 160 |
| 695 | < 5 | 320 | < 5 | 160 | <10 | 320 |
| 662 | 40 | NT | 20 | ⩾320 | 20 | 320 |
| 688 | 10 | 80 | 10 | 40 | 10 | 40 |
| 690 | 40 | 320 | 20 | 160 | 10 | ⩾320 |
| 700 | 20 | 80 | 5 | 80 | 10 | 80 |
| 701 | 10 | 80 | 10 | 40 | 10 | 40 |
| 702 | 10 | 640 | 5 | 320 | 10 | ⩾320 |
| 704 | 10 | 10 | 10 | 10 | 10 | 10 |
| 709 | 20 | 160 | 10 | 80 | 20 | 160 |

»NT« indique »non testé«.

Tableau III

Résumé des résultats de séroconversion pour les souches A/Hong Kong/117/77, C.N.C.M. I-099 et A/Brazil/11/78 après une dose de C.N.C.M. I-099

| Taux prévaccination | Résultats de séroconversion | | |
|---|---|---|---|
| | A/HK/117/77 | CNCM N° 1-099 | A/Brazil/11/78 |
| <5 (<10 pour A/Brazil/11/78) | 4/6 | 8 (9)/11 | 9/11 |
| 5/10 | 9/11 | 8/9 | 8/9 |
| ⩾20 | 4/4 | 2/2 | 2/2 |
| Total | 17/21$^{x}$) | 18(19)/22$^{xx}$) | 19/22 |
| Moyenne géométrique des taux*) (Pre- et post-vaccination) | 6/67 | 3/56 | 3/65 |

»*«   les taux inférieurs à 10 ont été notés comme zéro pour les calculs.
»$^{x}$«   les sérums d'un vacciné (le N° 662) n'ont pas été testés pour la souche A/Hong Kong/117/77.
»$^{xx}$«   un résultat est à la limite du significatif.

### 3. Réactions cliniques

Les symptômes notés étaient soit locaux soit déjà présents le jour de la vaccination. On a noté très peu de réactions locales sévères. Des réactions systématiques (élévation de température) ont été notées chez deux volontaires mais la température n'a toutefois pas dépassé 37,6° C.

### 4. Conclusion

Sur base des résultats ci-dessus, on peut conclure que le vaccin testé est sûr et fortement immunogène et que son profil d'excrétion est acceptable.

**Revendications pour les Etats contractants: BE, CH, DE, FR, GB, IT, LI, LU, NL, SE**

1. Procédé de préparation d'une souche recombinante de virus grippal de type A utilisable pour la préparation d'un vaccin antigrippal, par recombinaison d'une souche atténuée de virus grippal de type A avec une souche pathogène également de type A et isolement d'une souche recombinante dont le sérotype est celui de la souche pathogène parente, caractérisé en ce que la progéniture résultant de la recombinaison et présentant le sérotype de la souche pathogène est clonée, qu'on analyse les clones au point de vue du réassortiment de leurs gènes codant pour les polymérases (P) et qu'on isole comme souche vaccinale un recombinant dont les gènes codant pour les polymérases (P) ne sont pas tous issus du même virus parent.

2. Procédé suivant la revendication 1 caractérisé en ce que la souche atténuée de virus grippal de type A est la souche A/PR/8/34.

3. Procédé suivant la revendication 1 caractérisé en ce que la souche pathogène est la souche A/California/10/78.

4. La souche de virus grippal de type A déposée dans la Collection Nationale de Cultures de Microorganismes de l'Institut Pasteur à Paris sous le numéro C.N.C.M. I-099.

5. Vaccin antigrippal caractérisé en ce qu'il contient la souche de virus grippal suivant la revendication 4.

6. Vaccin antigrippal suivant la revendication 5 caractérisé en ce qu'il est présenté sous forme lyophilisée avec un diluant pharmaceutique pour administration nasale.

**Revendications pour l'Etat contractant: AT**

1. Procédé de préparation d'une souche recombinante de virus grippal de type A utilisable pour la préparation d'un vaccin antigrippal, par recombinaison d'une souche atténuée de virus grippal de type A avec une souche pathogène également de type A et isolement d'une souche recombinante dont le sérotype est celui de la souche pathogène parente, caractérisé en ce que la progéniture résultant de la recombinaison et présentant le sérotype de la souche pathogène est clonée, qu'on analyse les clones au point de vue du réassortiment de leurs gènes codant pour les polymérases (P) et qu'on isole comme souche vaccinale un recombinant dont les gènes codant pour les polymérases (P) ne sont pas tous issus du même virus parent.

2. Procédé suivant la revendication 1 caractérisé en ce que la souche atténuée de virus grippal de type A est la souche A/PR/8/34.

3. Procédé suivant la revendication 1 caractérisé en ce que la souche pathogène est la souche A/California/10/78.

## Patentansprüche für die Vertragsstaaten: BE, CH, FR, DE, GB, IT, LI, LU, NL, SE

1. Verfahren zur Herstellung eines rekombinanten Influenzavirusstammes des Typs A, der zur Herstellung eines Grippeimpfstoffs verwendbar ist, durch Rekombination eines attenuierten Influenzavirusstammes des Typs A mit einem pathogenen Stamm ebenfalls des Typs A und Gewinnung eines rekombinanten Stammes, dessen Serotyp derjenige des pathogenen Elternstammes ist, dadurch gekennzeichnet, daß man die aus der Rekombination hervorgehende und den Serotyp des pathogenen Stamms aufweisende Nachkommenschaft kloniert, die Klone auf die Anordnung ihrer für die Polymerasen (P) kodierenden Gene analysiert und als Impfstamm eine Rekombinante isoliert, deren für die Polymerasen (P) kodierende Gene nicht alle vom gleichen Elternvirus stammen.

2. Verfahren nach Anspruch 1, gekennzeichnet dadurch, daß der attenuierte Unfluenzavirusstamm des Typs A der Stamm A/PR/8/34 ist.

3. Verfahren nach Anspruch 1, gekennzeichnet dadurch, daß der pathogene Stamm der Stamm A/California/10/78 ist.

4. Influenzavirusstamm des Typs A, hinterlegt bei der Collection Nationale de Cultures de Microorganismes des Institut Pasteur in Paris unter der Nr. C.N.C.M. I-099.

5. Grippeimpfstoff, gekennzeichnet dadurch, daß er den Influenzavirusstamm gemäß Anspruch 4 enthält.

6. Grippeimpfstoff nach Anspruch 5, gekennzeichnet dadurch, daß er in lyophilisierter Form mit einem pharmazeutischen Verdünnungsmittel für nasale Gabe dargereicht wird.

## Patentansprüche für den Vertragsstaat: AT

1. Verfahren zur Herstellung eines rekombinanten Influenzavirusstammes des Typs A, der zur Herstellung eines Grippeimpfstoffs verwendbar ist, durch Rekombination eines attenuierten Influenzavirusstammes des Typs A mit einem pathogenen Stamm ebenfalls des Typs A und Gewinnung eines rekombinanten Stammes, dessen Serotyp derjenige des pathogenen Elternstammes ist, dadurch gekennzeichnet, daß man die aus der Rekombination hervorgehende und den Serotyp des pathogenen Stamms aufweisende Nachkommenschaft kloniert, die Klone auf die Anordnung ihrer für die Polymerasen (P) kodierenden Gene analysiert und als Impfstamm eine Rekombination isoliert, deren für die Polymerasen (P) kodierende Gene nicht alle vom gleichen Elternvirus stammen.

2. Verfahren nach Anspruch 1, gekennzeichnet dadurch, daß der attenuierte Influenzavirusstamm des Typs A der Stamm A/PR/8/34 ist.

3. Verfahren nach Anspruch 1, gekennzeichnet dadurch, daß der pathogene Stamm der Stamm A/California/10/78 ist.

## Claims for the Contracting States: BE, CH, DE, FR, GB, IT, LI, LU, NL, SE

1. Process for the preparation of a recombinant strain of influenza type A virus useful for the preparation of an influenza vaccine, by recombination of an attenuated strain of influenza type A virus with a pathogenic strain also of type A and isolation of a recombinant strain the serotype of which is the one of the pathogenic parent strain, characterised in that the progeny resulting from the recombination and having the serotype of the pathogenic strain is cloned, the clones are analysed for the reassortment of their genes coding for the polymerases (P) and there is isolated as vaccinal strain a recombinant whose genes coding for the polymerases (P) are not all issued from the same parent virus.

2. Process according to claim 1, characterised in that the attenuated influenza type A virus strain is the A/PR/8/34 strain.

3. Process according to claim 1, characterised in that the pathogenic strain is the A/California/10/78 strain.

4. The influenza type A virus strain deposited with the Collection Nationale de Cultures de Microorganismes de l'Institut Pasteur in Paris under C.N.C.M. No I-099.

5. Influenza vaccine characterised in that it comprises the influenza virus strain according to claim 4.

6. Influenza vaccine according to claim 5 characterised in that it is presented under freeze-dried form with a pharmaceutical diluent for nasal administration.

## Claims for the Contracting State: AT

1. Process for the preparation of a recombinant strain of influenza type A virus useful for the preparation of an influenza vaccine, by recombination of an attenuated strain of influenza type A virus with a pathogenic strain also of type A and isolation of a recombinant strain the serotype of which is the one of the pathogenic parent strain, characterized in that the progeny resulting from the recombination and having the serotype of the pathogenic strain is cloned, the clones are analysed for the reassortment of their genes coding for the polymerases (P) and there is isolated as vaccinal strain a recombinant whose genes coding for the polymerases (P) are not all issued from the same parent virus.

2. Process according to claim 1, characterised in that the attenuated influenza type A virus strain is the A/PR/8/34 strain.

3. Process according to claim 1, characterised in that the pathogenic strain is the A/California/10/78 strain.